# EUROPEAN PATENT APPLICATION

(11) **EP 2 458 353 A1**
(43) Date of publication of application: **30.05.2012**
(21) Application number: 10802059.5
(22) Date of filing: 12.07.2010
(51) Int. Cl.: G01K 1/02, G01K 7/00, G01K 7/01, G08C 17/00, G08C 19/00

(54) **BODY TEMPERATURE MEASURING SYSTEM AND DATA READING DEVICE AS WELL AS RELEVANT DRIVE AND CONTROL METHOD**

(30) Priority: 23.07.2009 JP 2009172563
(71) Applicant: Terumo Kabushiki Kaisha, Tokyo 151-0072 (JP)
(72) Inventor: OZAWA, Hitoshi, Tokyo 100-0005 (JP)
(74) Representative: TBK
(86) International application number: PCT/JP2010/004511
(87) International publication number: WO 2011/010437

(57) **Abstract**

In a body temperature measuring system, body temperature measurement with a temperature resolution of 0.01°C is implemented, and power consumption of a data reading device is reduced. A clinical thermometer of this invention is a body temperature measuring system including a body temperature tag (113) and a data reading device (101). A processing unit (115) of the body temperature tag (113) includes a power supply circuit, a semiconductor temperature sensor for detecting a band gap voltage, and a storage means for storing calibration data to calibrate the detected band gap voltage, and is configured to, upon activating the power supply circuit, send the detected band gap voltage via the antenna unit (114) together with the calibration data. The data reading device (101) includes an excitation means, and a sensing means for sensing a change in a magnetic field generated by excitation. The power level of the excitation means is changed upon sensing the change in the magnetic field.

## Description

### TECHNICAL FIELD

The present invention relates to a body temperature measuring system including a clinical thermometer for measuring the body temperature of an object and a data reading device for reading data from the clinical thermometer. In particular, the present invention relates to a data reading device for reading data from a clinical thermometer having a body temperature tag with excellent measurement accuracy in the body temperature measurement range of 32°C to 42°C and a driving control method thereof.

### BACKGROUND ART

In a hospital or the like, conventionally, the body temperature of a patient is periodically measured, and the measurement result is managed. When measuring the body temperature, in general, the object attaches a clinical thermometer to the measurement part and maintains it at rest for a predetermined time until completion of the measurement. When the measurement is completed, the measurer performs an operation of confirming and recording the measurement result.

However, it is difficult for a child or a seriously ill person to keep the clinical thermometer attached to the measurement part, and accurate body temperature measurement is not easy. In addition, the operation of confirming and recording the measurement result is a heavy load for the measurer. Hence, there is demanded an arrangement capable of recording without giving the measurer trouble.

For example, PTL1 proposes an adhesive clinical thermometer with an antenna. According to PTL1, the clinical thermometer is configured to operate upon receiving power supplied from an RF-ID reader/writer. The clinical thermometer does not have to incorporate a power supply, making it compact and lightweight. It is consequently possible to keep the clinical thermometer adhered to the measurement part of the object for a long time.

In addition, the measurement result can be read only by bringing the RF-ID reader/writer closer to the measurement part with the clinical thermometer adhered. This allows to largely reduce the load of the confirming/recording operation by the measurer.

### CITATION LIST

### PATENT LITERATURE

PTL1: Japanese Patent Laid-Open No. 2003-270051

### SUMMARY OF INVENTION

### TECHNICAL PROBLEM

The clinical thermometer described in PTL1 uses a thermistor as the temperature sensor. In general, the thermistor is compact, lightweight, and inexpensive but simultaneously has drawbacks such as a nonlinear temperature characteristic and susceptibility to aging and noise. Hence, the measurement accuracy is limited. To implement more accurate body temperature measurement (for example, body temperature measurement that requires an accuracy of ±0.05°C), a temperature sensor having a high measurement accuracy is preferably used.

In the clinical thermometer described in PTL1, the RF-ID reader/writer can always supply power to the clinical thermometer during the reading operation by the measurer. For this reason, when, for example, the clinical thermometers are adhered to a plurality of measurement parts of an object, or the measurer cannot immediately find the measurement part of the object, power consumption of the RF-ID reader/writer increases.

The present invention has been made in consideration of the above-described problems, and has as its object to, in a body temperature measuring system including an adhesive clinical thermometer with an antenna and a data reading device for reading data from the clinical thermometer, implement body temperature measurement with a temperature resolution of 0.01°C and reduce the power consumption of the data reading device.

### SOLUTION TO PROBLEM

In order to achieve the above-described object, a body temperature measuring system according to the present invention has the following arrangement. That is, a body temperature measuring system including a body temperature tag including an antenna unit and a processing unit, and a reading device for reading data from the body temperature tag, **characterized in that** the processing unit of the body temperature tag comprises: a power supply circuit connected to the antenna unit to be activated in accordance with generation of an induced electromotive force in the antenna unit; detection means in which at least two semiconductor temperature sensors are connected parallel to each other, each of the semiconductor temperature sensors being formed by connecting two types of semiconductors including a p-type semiconductor and an n-type semiconductor and detecting a band gap voltage generated when a current is supplied to a connection portion between the two types of semiconductors; and storage means for storing calibration data to calibrate the band gap voltage detected by the detection means, and is configured to, upon activating the power supply circuit, send the band gap voltage detected by the detection means to the reading device via the antenna unit together with the calibration data, the reading device comprises: excitation means capable of exciting at a predetermined power level; and sensing means for, when a magnetic field generated by excitation at a first power level by the excitation means has changed due to an influence of the antenna unit, sensing the change in the magnetic field, and the excitation means is configured to, when the sensing means senses the change in the magnetic field, excite at a second power level that allows to generate, in the antenna unit, the induced electromotive force to activate the power supply circuit.

### ADVANTAGEOUS EFFECTS OF INVENTION

According to the present invention, it is possible to, in a body temperature measuring system including an adhesive clinical thermometer with an antenna and a data reading device for reading data from the clinical thermometer, implement body temperature measurement with a temperature resolution of 0.01°C and reduce the power consumption of the data reading device.

Other features and advantages of the present invention will be apparent from the following descriptions taken in conjunction with the accompanying drawings, in which like reference characters designate the same or similar parts throughout the figures thereof.

### BRIEF DESCRIPTION OF DRAWINGS

The accompanying drawings, which are incorporated in and constitute a part of the specification, illustrate embodiments of the invention and, together with the description, serve to explain the principles of the invention.
Fig. 1 is a view showing the outer appearance of a body temperature measuring system 100 including a clinical thermometer 110 and a data reading device 101;
Fig. 2 is a block diagram showing the functional arrangement of a body temperature tag 113 including an antenna 114 and a processing unit 115;
Fig. 3 is a block diagram showing the functional arrangement of the data reading device 101;
Fig. 4 is a sequence chart showing the procedure of body temperature measurement processing in the body temperature measuring system 100;
Fig. 5 is a flowchart showing the procedure of body temperature measurement processing in the data reading device 101;
Fig. 6 is a view for explaining the operation of the data reading device 101 at the time of body temperature measurement processing;
Fig. 7 is a graph showing the characteristic of a semiconductor temperature sensor;
Fig. 8 is a circuit diagram showing the circuit arrangement of a sensor unit 211;
Fig. 9 is a block diagram showing the circuit arrangement of a circuit unit 212;
Fig. 10 is a circuit diagram showing the circuit arrangement of an overheat preventing unit 201;
Fig. 11 is a view showing steps in the manufacture of the clinical thermometer 110;
Fig. 12 shows graphs for explaining the contents of body temperature data calculation processing of a signal processing unit 304;
Fig. 13 is a view showing the outer appearance of a body temperature measuring system 1300 including a clinical thermometer 1310 and a data reading device 1301;
Fig. 14 is a view showing a state in which a body temperature measuring unit 1340 of the clinical thermometer 1310 is attached to an axillary portion of an object 1401; and
Fig. 15 is a view showing steps in the manufacture of the clinical thermometer 1310.

### DESCRIPTION OF EMBODIMENTS

First, the outline of the embodiments of the present invention will be described. The clinical thermometer of each embodiment to be described below features using a semiconductor temperature sensor (a sensor that detects a band gap voltage generated at the junction between a p-type semiconductor and an n-type semiconductor in proportional to the temperature) as its temperature sensor.

The semiconductor temperature sensor is suitable for accurate body temperature measurement because of its linear temperature characteristic and robustness to aging and noise.

However, even when the thermistor used in PTL1 is simply replaced with the semiconductor temperature sensor, body temperature measurement with a temperature resolution as high as 0.01°C cannot be implemented. To apply, it is important to eliminate various kinds of factors that affect the body temperature measurement accuracy.

In each embodiment to be explained below, when applying a wireless tag (a tag having an RF-ID function) including the semiconductor temperature sensor to an adhesive clinical thermometer with an antenna, various kinds of factors that affect the body temperature measurement accuracy are eliminated, thereby implementing a desired accuracy.

Additionally, in each embodiment to be described below, a data reading device for supplying power to the clinical thermometer and reading data from the clinical thermometer is provided with a low-power excitation mode and a high-power excitation mode. During the time the measurer is searching for the portion with the clinical thermometer adhered, the device excites the antenna by low power. When supplying power and reading data after finding the adhesion portion, the device excites the antenna by high power. This is because power consumption of the data reading device can be reduced by this arrangement.

The embodiments of the present invention will be described below in detail with reference to the accompanying drawings. Note that the present invention is not limited to the following embodiments, and various modifications can be adopted.

### [First Embodiment]

### <1. Outer Appearance of Body Temperature Measuring System>

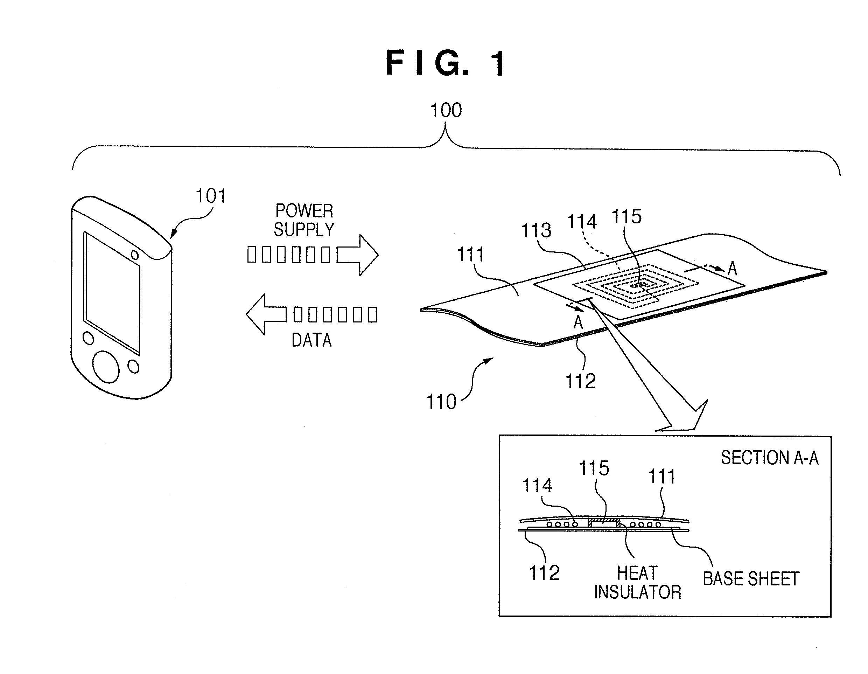

Fig. 1 is a view showing the outer appearance of a body temperature measuring system 100 including a clinical thermometer (an adhesive clinical thermometer with an antenna) 110 on which a wireless tag (RF-ID) including a semiconductor temperature sensor is arranged, and a data reading device 101 portable by a measurer.

As shown in Fig. 1, the clinical thermometer 110 includes a body temperature tag 113 serving as a wireless tag and sandwiched and fixed between an observe film 111 and a reserve film 112 (each film is semipermeable and has a thickness of about 100 µm).

The observe film 111 and the reserve film 112 can be obtained from materials including urethane-based polymers such as polyether polyurethane and polyester urethane, amide-based polymers such as a polyether polyamide block polymer, acrylic-based polymers such as polyacrylate, polyolefin-based polymers such as polyethylene, polypropylene, and an ethylene/vinyl acetate copolymer, and polyester-based polymers such as polyether polyester.

To prevent a skin surface with the film adhered from sweating or chlorosis, the reserve film 112 is preferably selected from materials having water vapor permeability. For example, using an urethane- or amide-based film is suitable. Note that each of the observe film 111 and the reserve film 112 can use one of the above-described materials or be a laminated film formed by laminating a plurality of films made of arbitrary materials.

The reserve film 112 has a thickness of 10 to 100 µm, and preferably, 20 to 40 µm to prevent any sense of incongruity when adhered to the skin surface. To make the film adhered to the skin surface excellently fit it, it is preferable to adjust the tensile strength to 100 to 900 kgf/cm² and the 100% modulus to about 10 to 100 kgf/cm². Using the reserve film 112 adjusted within this range is effective when adhered to the skin surface that moves largely.

From the viewpoint of preventing sweating, not only a non-porous film but also a porous film having water vapor permeability but no water permeability can effectively be used as the reserve film 112. Such a film can easily be obtained by a known porosification technique without particularly limiting the material.
As the noticeable tendency of a non-porous film, the thicker the film is, the lower the water vapor permeability is. However, a porous film is useful because the water vapor permeability does not conspicuously lower in proportion to the thickness.

An adhesive is applied to the reserve film 112 so that the clinical thermometer 110 can directly be adhered to an appropriate measurement part on the body surface of an object. The adhesive can be any material of normal medical grade. Examples are acrylic-based adhesives, polyurethane-based adhesives, or natural rubber- or synthetic rubber-based adhesives, and solvent-, water borne-, hot melt-, and dry blend-based adhesives mainly containing a medical polymer. If radiation sterilization and, more particularly, intensive gamma-ray sterilization is necessary, it is preferable to avoid use of an acrylic-based adhesive or a polyurethane-based adhesive because radiation rays may lower the adhesion.

If the area of the observe film 111 is larger than that of the reserve film 112, the adhesive region is left to adhere the clinical thermometer to the measurement part of the object, and an adhesive is applied to that region. Note that in this case, the adhesive can be any material of normal medical grade. Examples are acrylic-based adhesives, polyurethane-based adhesives, natural rubber- or synthetic rubber-based adhesives, and solvent-, water borne-, hot melt-, and dry blend-based adhesives mainly containing a medical polymer. If radiation sterilization and, more particularly, intensive gamma-ray sterilization is necessary, it is preferable to avoid use of an acrylic-based adhesive or a polyurethane-based adhesive because radiation rays may lower the adhesion.

In addition, the observe film 111 and the reserve film 112 are so flexible that they can deform conforming to the shape of the measurement part of the object when the clinical thermometer 110 is adhered to the measurement part. Since a processing unit 115 is fixed in tight contact with the measurement part, the clinical thermometer 110 can accurately detect the body temperature of the object.

The body temperature tag 113 that is a wireless tag having an RF-ID function and including a semiconductor temperature sensor includes an antenna coil (to be simply referred to as an antenna hereinafter) 114 and the processing unit 115 on a base sheet. The body temperature tag 113 receives power supply (for example, power supply by an induced electromotive force generated by an electromagnetic wave having a frequency of 13.56 MHz) from the data reading device 101 via the antenna 114. The entire processing unit 115 is activated by supplying the power to a power supply circuit (not shown) included in it. The processing unit 115 sends, as data, band gap voltage data (voltage data that correlates with the body temperature of the object) acquired by a temperature sensing unit including a semiconductor temperature sensor to be described later to the data reading device 101 via the antenna 114 together with various kinds of information.

Note that out of the antenna 114 and the processing unit 115 included in the body temperature tag 113, the processing unit 115 is covered by a heat insulator (for example, a heat insulator having a four-layer structure (nonwoven fabric + transparent polyethylene film + aluminum layer + polyethylene foam film) including an aluminum layer and formed into a thickness of about 1 mm) (see the section A-A). This enables to eliminate the influence of outside air temperature.

The data reading device 101 includes an RF-ID reader/writer. When approaching the body temperature tag 113, the data reading device 101 magnetically couples with it so as to supply power to the power supply circuit included in the processing unit 115 of the body temperature tag 113 and receive data from the body temperature tag 113.

As described above, in the body temperature measuring system 100, the clinical thermometer 110 is an adhesive clinical thermometer with an antenna, which operates upon receiving power supplied from the RF-ID reader/writer included in the data reading device. Since no internal power supply is necessary, the clinical thermometer can be compact and lightweight. It is consequently possible to keep the clinical thermometer adhered to the measurement part of the object for a long time.

The measurement result can be read only by placing the data reading device 101 including the RF-ID reader/writer that sends an electromagnetic wave having a predetermined frequency of, for example, 13.56 MHz at a distance of about 5 to 15 mm from the measurement part with the clinical thermometer 110 adhered. This allows to largely reduce the load of the measurement result confirming/recording operation by the measurer.

### <2. Functional Arrangement of Body Temperature Tag 113>

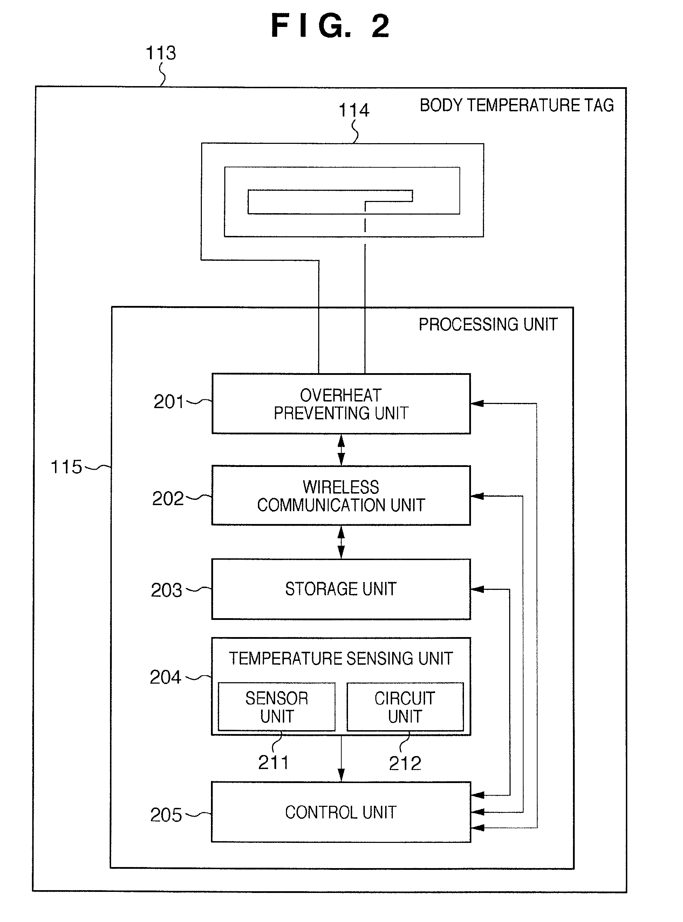

The functional arrangement of the body temperature tag 113 will be described next. Fig. 2 is a block diagram showing the functional arrangement of the body temperature tag 113 including the antenna 114 and the processing unit 115.

Referring to Fig. 2, an overheat preventing unit 201 controls to stop body temperature measurement processing when the body temperature tag 113 transits to the state that affects the accuracy of body temperature measurement. The state that affects the accuracy of body temperature measurement is, for example, the state in which excessive power is supplied from the data reading device 101 via the antenna 114, and the body temperature tag 113 itself generates heat (raises the temperature) to cause an error in the body temperature measurement. Note that the circuit arrangement of overheat preventing unit 201 will be described later in detail.

A wireless communication unit 202 includes a rectifying circuit, a boost circuit, and the like. The wireless communication unit 202 also functions as a power supply unit that converts the AC voltage generated by the antenna 114 into a predetermined DC voltage and supplies it to a storage unit 203 and a control unit 205. The wireless communication unit 202 also sends voltage data acquired by the control unit 205 to the data reading device 101 via the antenna 114 as data in a predetermined format together with various kinds of information.

The storage unit 203 stores the calibration data of the temperature sensing unit to be described later, identification information unique to the body temperature tag 113, and the like.

A temperature sensing unit 204 includes a sensor unit 211 with a semiconductor temperature sensor, and a circuit unit 212 that processes the output from the sensor unit 211. Note that the circuit arrangements of the sensor unit 211 and the circuit unit 212 will be described later in detail.

The control unit 205 controls the operations of the wireless communication unit 202 and the storage unit 203. The control unit 205 also processes the output from the temperature sensing unit 204 and sends it to the wireless communication unit 202 as voltage data. Note that a sufficient voltage is necessary (a voltage Vcc higher the voltage required for operating the storage unit 203 or the control unit 205 is necessary) for implementing accurate body temperature measurement, for example, a measurement accuracy of 0.01 °C to 0.05°C in the semiconductor temperature sensor applied to the sensor unit 211 of the temperature sensing unit 204. Hence, the control unit 205 includes a power supply circuit (boost means) for this purpose. This power supply circuit is activated in accordance with generation of an induced electromotive force in the antenna 114.

### <3. Functional Arrangement of Data Reading Device>

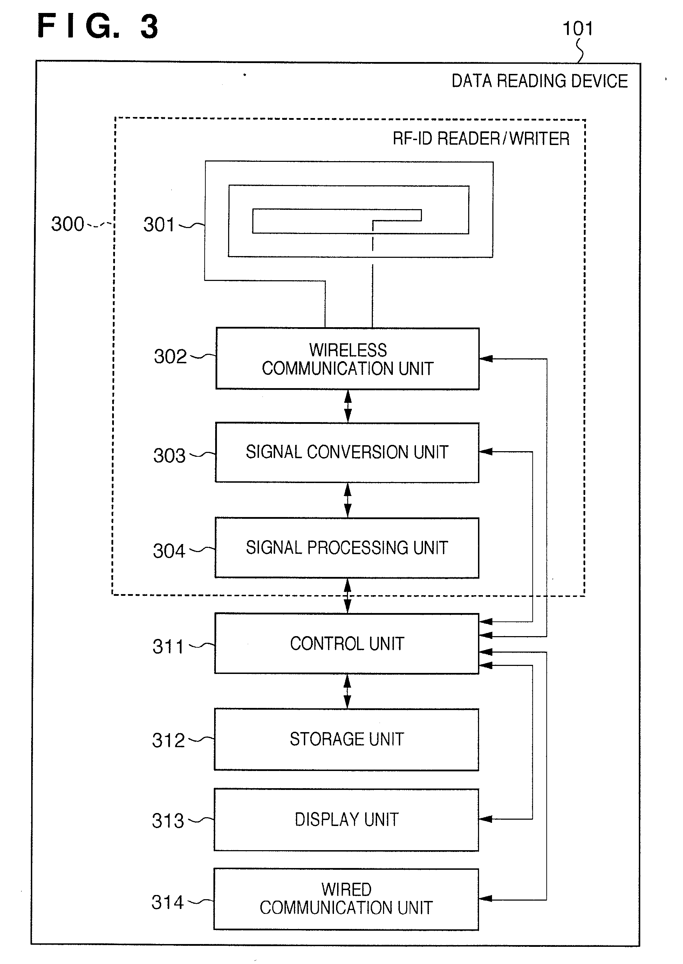

The functional arrangement of the data reading device 101 will be described next. Fig. 3 is a block diagram showing the functional arrangement of the data reading device 101. Although not illustrated, the data reading device 101 includes a power supply unit formed from a battery, a battery charger, or the like, and operation switches including a power switch, a select switch for selecting the measurement range, and a body temperature data reading start switch.

Referring to Fig. 3, an RF-ID reader/writer 300 includes an antenna 301, a wireless communication unit 302, a signal conversion unit 303, and a signal processing unit 304.

The antenna 301 generates an electromagnetic wave having a predetermined frequency of, for example, 13.56 MHz to detect the presence/absence of the antenna 114 of the body temperature tag 113, or electromagnetically couples with the antenna 114 of the body temperature tag 113 to supply power to the power supply circuit of the body temperature tag 113 or receive data from the body temperature tag 113.

The wireless communication unit 302 controls the voltage to be applied to the antenna 301 to detect the presence/absence of the antenna 114 of the body temperature tag 113 or supply power to the body temperature tag 113 via the antenna 301, or sends data received from the body temperature tag 113 via the antenna 301 to the signal conversion unit 303.

The signal conversion unit 303 converts the data sent from the wireless communication unit 302 into digital data and sends it to the signal processing unit 304.

The signal processing unit 304 processes the digital data received from the signal conversion unit 303 to calculate the body temperature. More specifically, the signal processing unit 304 calculates body temperature data based on voltage data and calibration data included in the received digital data. The signal processing unit 304 also sends the calculated body temperature data to a control unit 311 together with identification information contained in the received digital data.

The control unit 311 includes a CPU such as a microcomputer, a ROM that stores various kinds of data and the control program of the entire device to be executed by the CPU, and a RAM that serves as a work area and temporarily stores measured data and various kinds of data, and controls the operations of the wireless communication unit 302, the signal conversion unit 303, and the signal processing unit 304. The control unit 311 also stores body temperature data sent from the signal processing unit 304 in a storage unit 312 together with the identification information or displays the data on a display unit 313. In addition, the control unit 311 sends the body temperature data stored in the storage unit 312 to another information processing apparatus (another information processing apparatus connected by a cable via the wired communication unit 314) via a wired communication unit 314 together with the identification information.

### <4. Procedure of Body Temperature Measurement Processing>

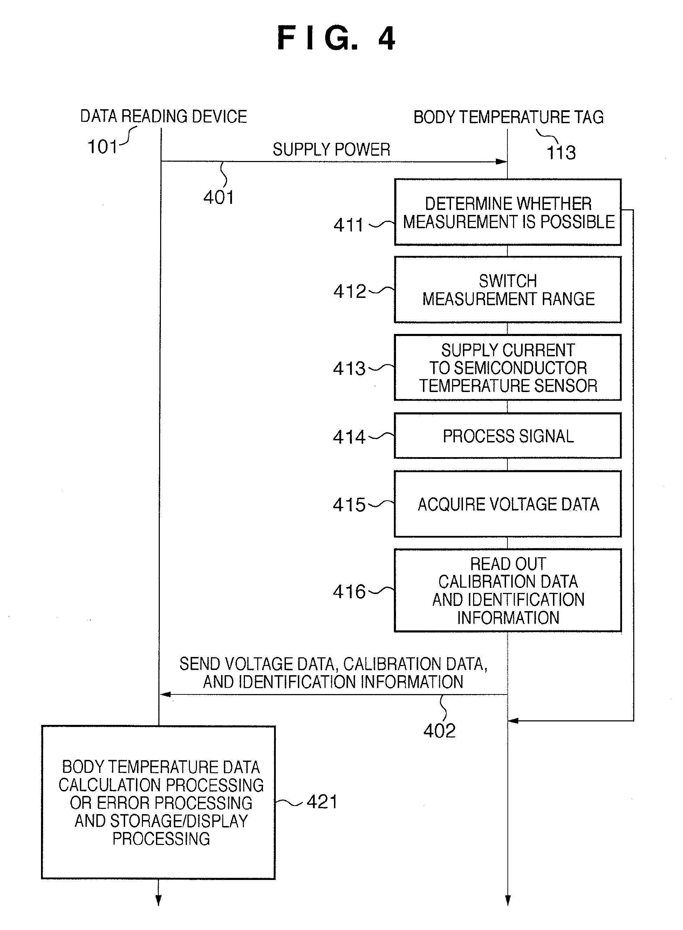

The procedure of body temperature measurement processing in the body temperature measuring system 100 will be explained next. Fig. 4 is a sequence chart showing the procedure of body temperature measurement processing in the body temperature measuring system 100.

As shown in Fig. 4, the data reading device 101 is activated to generate an electromagnetic wave having a predetermined frequency of, for example, 13.56 MHz at a predetermined power level (power level 1). In this state, the measurer (not shown) brings the data reading device 101 closer to the clinical thermometer 110 attached to an axillary portion that is one of appropriate body temperature measurement parts of an object (not shown). Upon sensing the antenna 114, the data reading device 101 raises the power level to power level 2. This allows the antennas 301 and 114 to electromagnetically couple with each other so that the data reading device 101 supplies power to the clinical thermometer 110 (401).

In the clinical thermometer 110 that has receives the power, the processing unit 115 is activated to determine whether the body temperature tag 113 is in the state that affects the body temperature measurement accuracy. Upon determining that the processing unit 115 is in the state that affects the body temperature measurement accuracy, the processing unit 115 does not perform the subsequent processing. In this case, the data reading device 101 determines that no data has been sent from the clinical thermometer 110 within a predetermined time from power supply, and performs display processing to display an error message on the display unit 313 (421).

On the other hand, upon determining that the body temperature tag 113 is not in the state that affects the body temperature measurement accuracy, the processing unit 115 starts processing.

More specifically, after switching to a preset measurement range (to be described later in detail) (412), the processing unit 115 supplies a current to the semiconductor temperature sensor in the sensor unit 211 and detects the band gap voltage (413).

Additionally, the circuit unit 212 processes the detected band gap voltage (414), and the control unit 205 acquires the voltage data (415).

The acquired voltage data is sent to the data reading device 101 together with calibration data and identification information stored in the storage unit 203 (402, 416).

The data reading device 101 calculates body temperature data based on the voltage data and the calibration data sent from the clinical thermometer 110. The data reading device 101 also stores the calculated body temperature data in the storage unit 312 in association with the identification information and displays the body temperature data on the display unit 313 (421).

### <5. Procedure of Body Temperature Measurement Processing in Data Reading Device>

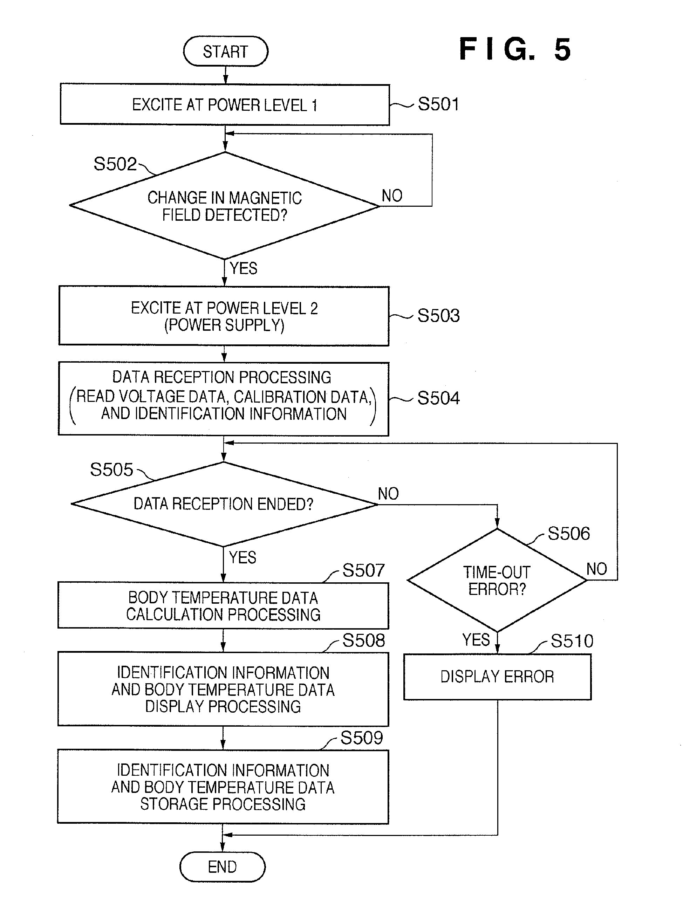

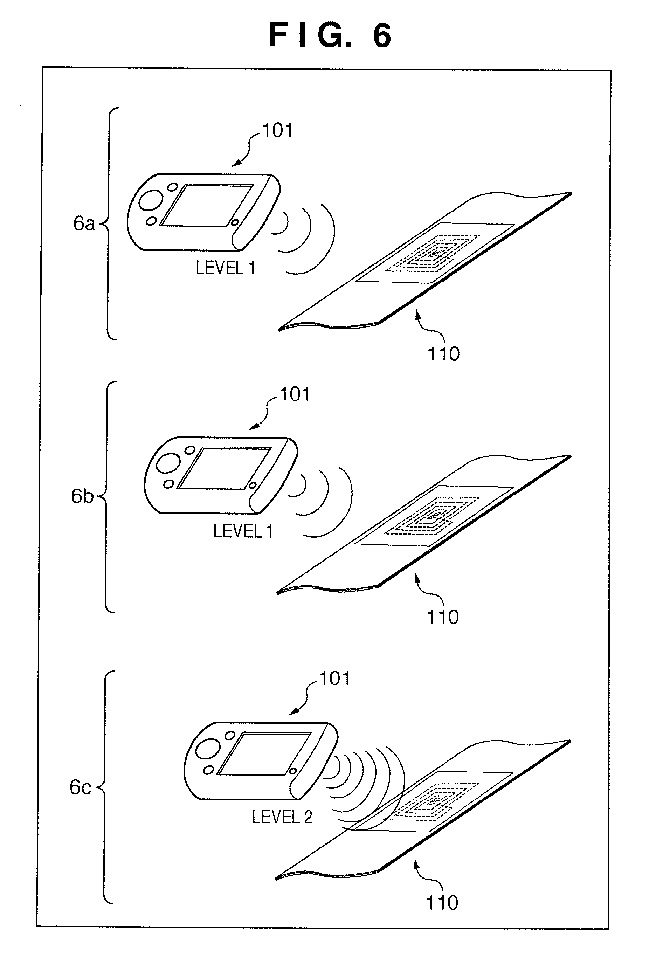

The operation of the data reading device 101 at the time of body temperature measurement processing in the body temperature measuring system 100 will be described next in detail with reference to Figs. 5 and 6. Fig. 5 is a flowchart showing the procedure of processing in the data reading device 101 at the time of body temperature measurement processing. Fig. 6 is a view for explaining the operation of the data reading device 101 at the time of body temperature measurement processing.

As shown in Fig. 5, when activated, the data reading device 101 excites the antenna 301 at power level 1 to generate an electromagnetic wave in step S501 (6a of Fig. 6). Note that the electromagnetic wave generated at this time has a frequency of, for example, 13.56 MHz.

In step S502, it is determined whether the magnetic field has changed due to generation of the electromagnetic wave from the antenna 301. If the antenna 114 of the body temperature tag 113 is out of the range of the generated magnetic field, as indicated by 6b of Fig. 6, the magnetic field does not change.

On the other hand, when the antenna 114 of the body temperature tag 113 enters the range of the generated magnetic field, as indicated by 6c of Fig. 6, the magnetic field changes. In this case, it is determined ins step S502 that the change in the magnetic field is sensed, and the process advances to step S503.

In step S503, the data reading device 101 excites the antenna 301 at power level 2. Power level 2 is higher than power level 1, and causes electromagnetic coupling between the antenna 301 and the antenna 114 of the body temperature tag 113 so that the antenna 114 generates an induced electromotive force suitable for activating the power supply circuit (to be described later in detail) included in the body temperature tag 113.

When the antenna 301 is excited at power level 2 to supply power to the body temperature tag 113 in step S503, the data reading device 101 starts receiving voltage data, calibration data, and identification information sent from the body temperature tag 113 in step S504.

In step S505, it is determined whether the reception of the voltage data, the calibration data, and the identification information has ended. Upon determining in step S505 that the reception of the voltage data, the calibration data, and the identification information has ended, the data reading device 101 calculates body temperature data based on the voltage data and the calibration data in step S507.

In step S508, the data reading device 101 displays the calculated body temperature data on the display unit 313 together with the identification information received in step S404. In step S509, the data reading device 101 stores the calculated body temperature data in the storage unit 312 together with the identification information, and ends the processing.

On the other hand, if it is determined in step S505 that the reception of the voltage data, the calibration data, and the identification information has not ended, the process advances to step S506 to determine whether a predetermined time has elapsed.

If it is determined in step S506 that the predetermined time has not elapsed, the process returns to step S505 to continue the processing of receiving the voltage data, the calibration data, and the identification information. Upon determining in step S506 that the predetermined time has elapsed, the data reading device 101 determines that a time-out error has occurred, and the process advances to step S510.

In step S510, the data reading device 101 determines that the voltage data, the calibration data, and the identification information have not correctly been received within the predetermined time, displays an error message on the display unit 313, and ends the processing.

Note that not explicitly illustrated in Fig. 5, the data reading device 101 excites the antenna 301 again at power level 1 when the processing has ended.

As described above, after activation, the data reading device 101 excites the antenna 301 at power level 1. After sensing of the antenna 301, the power level changes to power level 2. This arrangement allows to reduce the power consumption of the data reading device 101.

### <6. Description of Semiconductor Temperature Sensor>

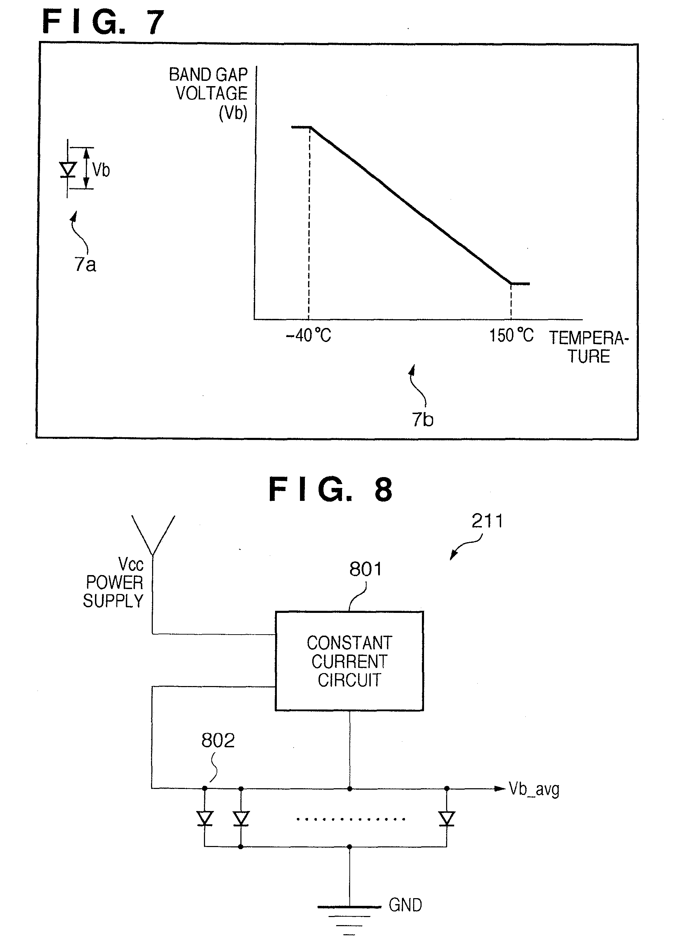

A general semiconductor temperature sensor applied to the sensor unit 211 will be described next. Fig. 7 is a graph showing the characteristic of the semiconductor temperature sensor. In this embodiment, the semiconductor temperature sensor applied to the sensor unit 211 is formed by connecting a p-type semiconductor and an n-type semiconductor, and detects a voltage (band gap voltage Vb) generated at the connection portion (junction) in correlation to the temperature when a DC current is supplied (7a of Fig. 7).

Note that in the semiconductor temperature sensor, the band gap voltage Vb and the temperature have a linearity within a wide range of about -40°C to +150°C, as indicated by 7b of Fig. 7. In addition, the semiconductor temperature sensor has robustness to aging and noise as compared to a thermistor.

### <7. Circuit Arrangement of Sensor Unit 211>

The circuit arrangement of the sensor unit 211 will be described next. Fig. 8 is a circuit diagram showing the circuit arrangement of the sensor unit 211 formed using the semiconductor temperature sensor indicated by 7a of Fig. 7.

Referring to Fig. 8, a constant current circuit 801 adjusts and uniforms the current to be supplied to each semiconductor temperature sensor based on the power Vcc supplied from the control unit 205.

Semiconductor temperature sensors 802 are connected in series with the constant current circuit 801 on its downstream side. Note that a plurality of, preferably, six to 10, and particularly preferably, eight semiconductor temperature sensors 802 are connected to the constant current circuit 801. The semiconductor temperature sensors are connected parallel to each other. As the number of semiconductor temperature sensors parallelly connected increases, the temperature resolution becomes higher, but the manufacturing cost increases. If the number of semiconductor temperature sensors is small, the temperature resolution lowers.

The plurality of semiconductor temperature sensors are connected parallelly to eliminate the influence of the individual difference between them. To implement accurate body temperature measurement, the influence of the individual difference between the semiconductor temperature sensors cannot be neglected. In the sensor unit 211, a plurality of, particularly preferably, eight to 10 semiconductor temperature sensors are connected parallelly to obtain an average value, thereby eliminating the influence of the individual difference and obtaining a temperature resolution of 0.01°C. With this arrangement, a measurement accuracy within 0.05°C is obtained.

For this reason, the sensor unit 211 outputs an average value Vb_avg of voltages Vb1, Vb2,..., Vbn output from the semiconductor temperature sensors.

The current may be supplied to each semiconductor temperature sensor not once but a plurality of number of times. In this case, the sensor unit 211 outputs the voltage Vb_avg a plurality of number of times.

### <8. Circuit Arrangement of Circuit Unit 212>

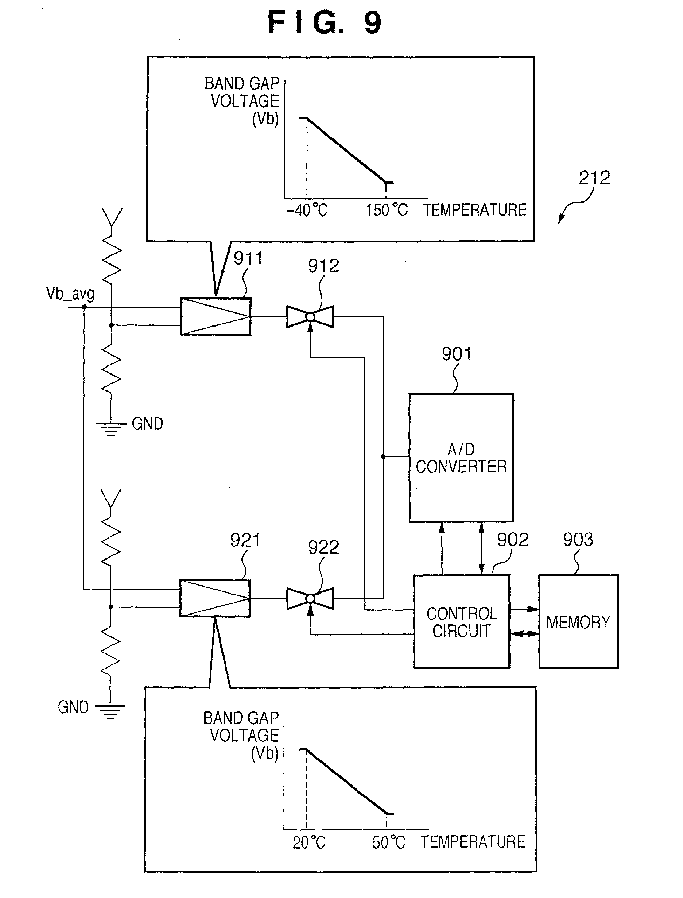

The circuit arrangement of the circuit unit 212 will be described next. Fig. 9 is a block diagram showing the circuit arrangement of the circuit unit 212.

As shown in Fig. 9, the circuit unit 212 includes two systems, that is, a system connected to an A/D converter 901 via a comparator/amplifier 911 and an analog switch 912 and a system connected to the A/D converter 901 via a comparator/amplifier 921 and an analog switch 922.

The former system (first system) inputs the voltage Vb_avg output from the sensor unit 211 to the A/D converter 901 within the measurement range of -40°C to +150°C. The latter system (second system) inputs the voltage Vb_avg output from the sensor unit 211 to the A/D converter 901 within the measurement range of 20°C to 50°C.

Which one of the first system and the second system is used to output (that is, measurement range) is instructed by selecting the measurement range using the select switch (not shown) of the data reading device 101, and controlled by switching the analog switches 912 and 922 based on a signal from a control circuit 902. To measure the body temperature at a higher accuracy, that is, with a temperature resolution of 0.01°C at a measurement accuracy within 0.05°C, the second system is selected.

The voltage Vb_avg input to the A/D converter 901 is A/D-converted by the A/D converter 901 and input to the control circuit 902 as digital data.

The digital data input to the control circuit 902 is sent to the wireless communication unit 202.

Note that when the sensor unit 211 outputs the voltage Vb_avg a plurality of number of times, each digital data may temporarily be stored in a memory 903, and after the control circuit 902 has calculated the average value of all digital data stored in the memory 903, sent to the wireless communication unit 202.

### <9. Circuit Arrangement of Overheat Preventing Unit>

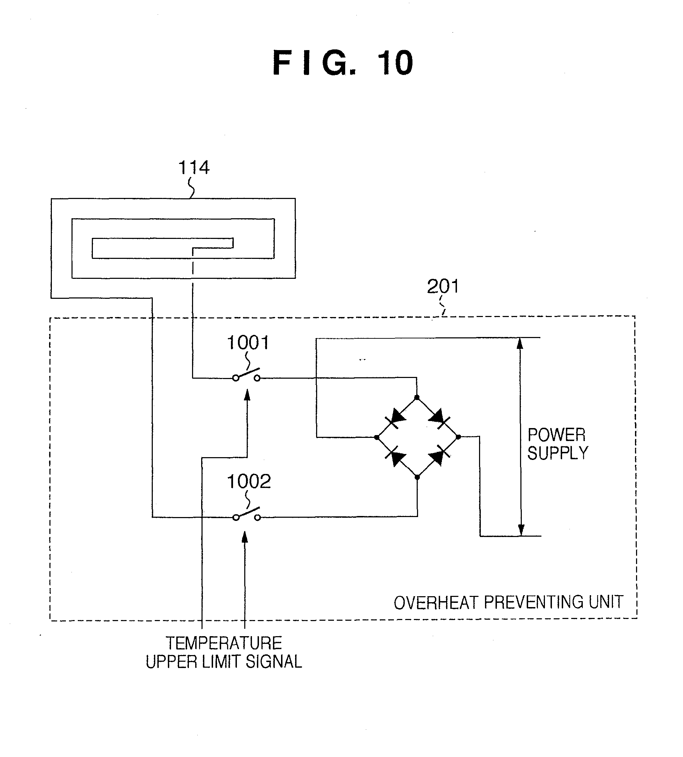

The circuit arrangement of the overheat preventing unit 201 will be described next. Fig. 10 is a circuit diagram showing the circuit arrangement of the overheat preventing unit 201.

Referring to Fig. 10, upon receiving a temperature upper limit signal from the control unit 205, switches 1001 and 1002 stop supplying power to the processing unit 115 and also stop sending data to the data reading device 101.

When the value of digital data calculated by the control unit 205 is equal to or smaller than a predetermined value, the control unit determines that the body temperature tag is in the state that affects the body temperature measurement accuracy, and outputs the temperature upper limit signal. This is because when excessive power is supplied from the RF-ID reader/writer, the entire body temperature tag 113 generates heat to make it impossible to accurately measure the body temperature, as described above.

The processing unit 115 thus stops the processing when the body temperature tag transits to the state that affects the accuracy of body temperature measurement. This allows the data reading device 101 to prevent display of a wrong measurement result.

### <10. Steps in Manufacture of Clinical Thermometer 110>

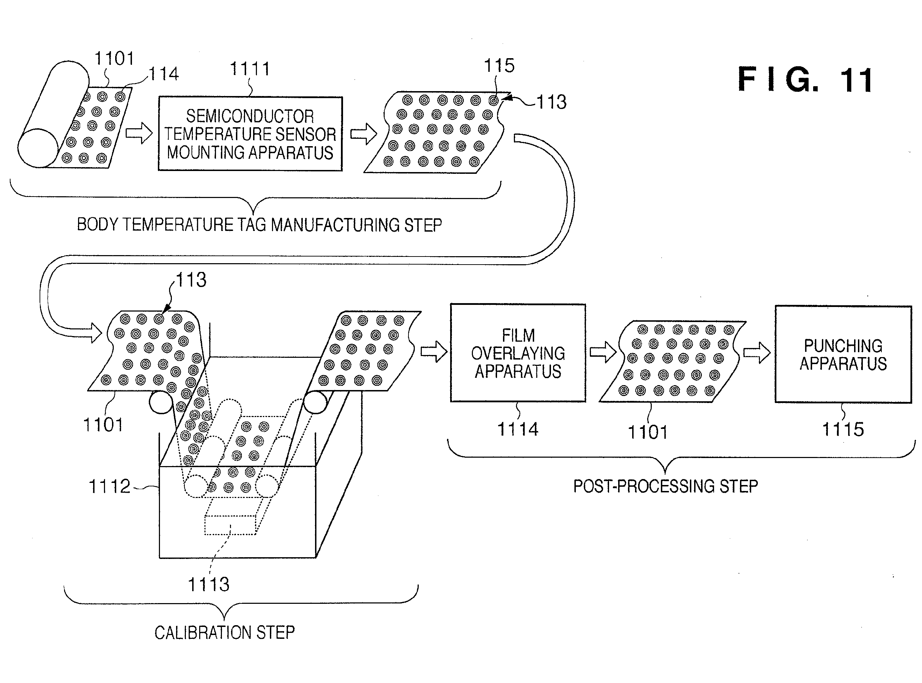

Steps in the manufacture of the clinical thermometer 110 will be described next. Fig. 11 is a view showing the steps in the manufacture of the clinical thermometer 110.

As shown in Fig. 11, the manufacturing steps of the clinical thermometer 110 can roughly be divided into a body temperature tag manufacturing step, a calibration step, and a post-processing step.

In the body temperature tag manufacturing step, a strip-shaped base sheet 1101 on which a plurality of antennas 114 are arranged is sequentially conveyed to a semiconductor temperature sensor mounting apparatus 1111 to electrically connect the processing unit 115 to each antenna 114, thereby forming the body temperature tags 113.

In the calibration step, the strip-shaped base sheet 1101 on which the plurality of body temperature tags 113 are arranged is sequentially conveyed to a thermostatic chamber 1112. The thermostatic chamber 1112 is a chamber managed to a preset temperature, for example, 37°C.

An RF-ID reader/writer 1113 is arranged inside the thermostatic chamber 1112. The RF-ID reader/writer 1113 communicates with each body temperature tag 113 using an electromagnetic wave having a predetermined frequency of, for example, 13.56 MHz when each body temperature tag 113 passes above the RF-ID reader/writer 1113.

More specifically, the RF-ID reader/writer 1113 receives band gap voltage data of each body temperature tag 113 and writes the received band gap voltage data in the storage unit 203 of the body temperature tag 113 as calibration data together with the temperature of the thermostatic chamber 1112. Note that the thermostatic chamber 1112 is assumed to be designed to sufficiently slowly convey the base sheet 1101 so that the body temperature tag 113 that has transited to the equilibrium state upon receiving the temperature of the thermostatic chamber 1112 passes on the RF-ID reader/writer 1113.

Note that although only one thermostatic chamber 1112 is arranged in the example shown in Fig. 11, the number of thermostatic chambers 1112 need not always be one. A plurality of thermostatic chambers set to different temperatures, for example, 32°C and 42°C, or 32°C, 36°C, and 42°C may be prepared, and calibration data for each temperature may be written in each body temperature tag 113.

When a plurality of thermostatic chambers are prepared, one of the thermostatic chambers may be used for inspection of the body temperature tags 113. More specifically, the body temperature tags 113 in which the calibration data has been written are conveyed to a thermostatic chamber (thermostatic chamber for inspection) managed to a preset temperature to receive the band gap voltage data and the calibration data from each body temperature tag 113. The temperature calculated based on the voltage data and the calibration data is compared with that of the thermostatic chamber. It is thus determined whether the temperature falls within a predetermined error range.

In the post-processing step, the base sheet 1101 on which the plurality of body temperature tags 113 with the calibration data written are arranged is sequentially conveyed to a film overlaying apparatus 1114. The film overlaying apparatus 1114 covers the processing unit 115 of each body temperature tag 113 with a heat insulator (for example, a heat insulator having a four-layer structure (nonwoven fabric + transparent polyethylene film + aluminum layer + polyethylene foam film) including an aluminum layer and formed into a thickness of about 1 mm). In addition, the films 111 and 112 (each film is semipermeable and has a thickness of about 100 µm) are bonded to the upper and lower surfaces of the base sheet 1101 using an adhesive. The above-described adhesive is applied to the reserve film 112.

The base sheet 1101 to which the films are bonded by the film overlaying apparatus 1114 is conveyed to a punching apparatus 1115 and cut for each body temperature tag 113, thereby forming the clinical thermometer 110.

### <11. Body Temperature Data Calculation Processing in Data Reading Device>

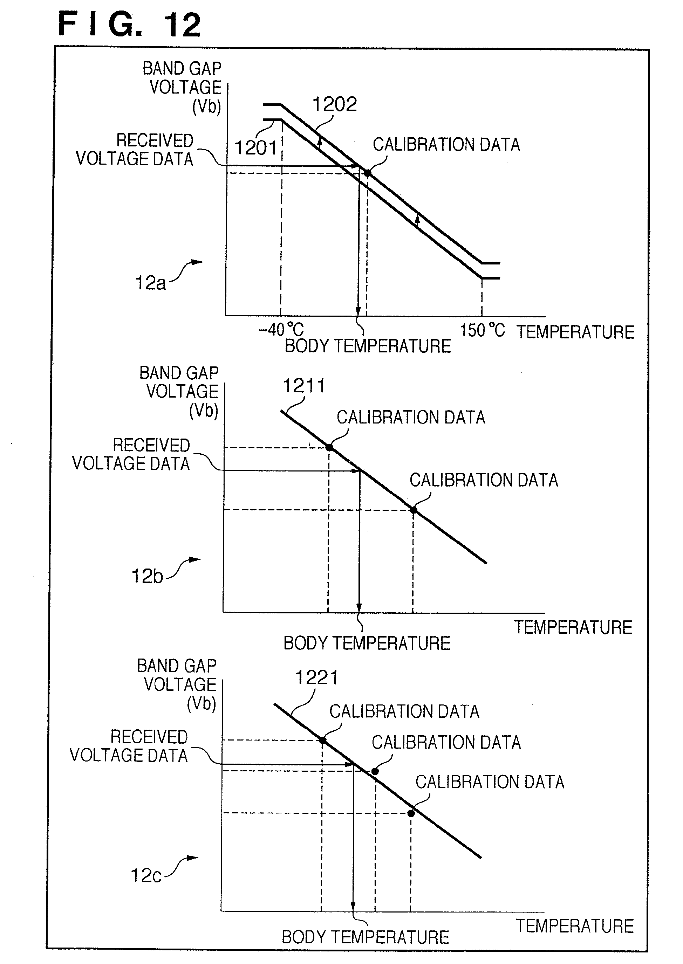

Processing of causing the signal processing unit 304 of the data reading device 101 to calculate body temperature data will be described next. Fig. 12 shows graphs for explaining the contents of body temperature data calculation processing of the signal processing unit 304.

The signal processing unit 304 corrects, based on calibration data, the graph (function) representing the correspondence between band gap voltage data and body temperature data in a semiconductor temperature sensor serving as a reference, and then substitutes the received band gap voltage data to derive the body temperature data.

In Fig. 12, 12a represents a view showing correction processing when one type of calibration data corresponding to one type of temperature is received. As indicated by 12a of Fig. 12, upon receiving one type of calibration data corresponding to one type of temperature, the offset value of the correspondence between band gap voltage data and body temperature data in the semiconductor temperature sensor serving as a reference is adjusted. More specifically, a graph 1201 is wholly translated in the direction of the arrows to obtain a graph 1202.

The signal processing unit 304 substitutes voltage data received from the clinical thermometer 110 into the graph 1202 after the translation, thereby deriving body temperature data.

In Fig. 12, 12b represents a view showing correction processing when two types of calibration data corresponding to two types of temperatures are received. As indicated by 12b of Fig. 12, upon receiving two types of calibration data corresponding to two types of temperatures, a line 1211 passing through the two points is calculated as a graph representing the correspondence between band gap voltage data and body temperature data in the semiconductor temperature sensor.

The signal processing unit 304 substitutes band gap voltage data received from the clinical thermometer 110 into the calculated line, thereby deriving body temperature data.

In Fig. 12, 12c represents a view showing correction processing when three or more types of calibration data corresponding to three or more types of temperatures are received. As indicated by 12c of Fig. 12, upon receiving three or more types of calibration data corresponding to three or more types of temperatures, a regression line 1221 is calculated by the least squares method based on the three or more points as a graph representing the correspondence between band gap voltage data and body temperature data in the semiconductor temperature sensor.

The signal processing unit 304 substitutes band gap voltage data received from the clinical thermometer 110 into the calculated regression line 1221, thereby calculating body temperature data.

As is apparent from the above description, the clinical thermometer according to this embodiment is an adhesive clinical thermometer with an antenna to which a semiconductor temperature sensor is applied.

When applying the semiconductor temperature sensor:
the processing unit is covered with a heat insulator to eliminate the influence of outside air temperature;
to eliminate the influence of the individual difference between semiconductor temperature sensors, the plurality of semiconductor temperature sensors are connected parallelly in the sensor unit;
to eliminate measurement errors, the current is supplied to the sensor unit a plurality of number of times in one cycle of body temperature measurement, and the average value is output;
to eliminate the influence of the individual difference between body temperature tags, calibration data is stored for each body temperature tag in the storage unit of the body temperature tag. When sending voltage data, the data reading device sends the calibration data together; and
· if a measurement error may occur due to heat generated by the body temperature tag, the overheat preventing unit stops body temperature measurement to prevent any wrong measurement result from being displayed on the data reading device.

It is therefore possible to implement accurate body temperature measurement at a measurement accuracy within 0.05°C especially by obtaining a temperature resolution of 0.01°C at 32°C to 42°C that is the general measurement range of human body temperature measurement.

Additionally, the data reading device according to this embodiment switches between two power levels when exciting the antenna.

More specifically,
· the antenna is excited at power level 1 necessary for sensing until the adhesive clinical thermometer is sensed;
· after the adhesive clinical thermometer is sensed, the antenna is excited at power level 2 for generating an induced electromotive force suitable for activating the power supply circuit of the clinical thermometer; and after power supply to the adhesive clinical thermometer and reception of various kinds of data for the adhesive clinical thermometer are completed, the antenna is excited at power level 1 again.

This enables to reduce the power consumption of the data reading device.

### [Second Embodiment]

In the first embodiment, the processing unit is arranged in the antenna. However, the present invention is not limited to this, and the processing unit may be connected to the antenna via a conductor running from the antenna. A body temperature measuring system according to this embodiment will be described below. Note that the difference from the first embodiment will mainly be explained for the sake of simplicity.

### <1. Outer Appearance of Body Temperature Measuring System>

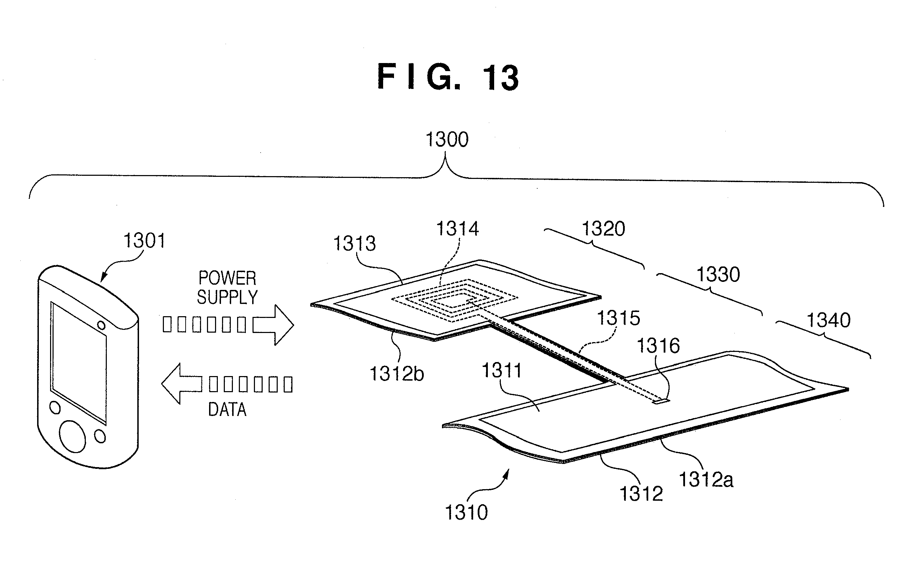

Fig. 13 is a view showing the outer appearance of a body temperature measuring system 1300 according to the second embodiment of the present invention, which includes a clinical thermometer (an adhesive clinical thermometer with an antenna) 1310 on which a wireless tag (RF-ID) including a semiconductor temperature sensor is arranged, and a data reading device 1301 portable by a measurer.

As shown in Fig. 13, the clinical thermometer 1310 can be divided into three parts in terms of function. The first part is an antenna unit 1320 including an antenna 1314. The second part is a running portion 1330 in which a conductor 1315 for electrically connecting the antenna 1314 and a processing unit 1316 is arranged. The third part is a body temperature measuring unit 1340 including the processing unit 1316.

The antenna 1314 has the same arrangement and functions as those of the antenna 114 described in the first embodiment. The processing unit 1316 has the same arrangement and functions as those of the processing unit 115 described in the first embodiment. The body temperature measuring unit 1340 has the same arrangement and functions as those of the processing unit 115 described in the first embodiment. The data reading device 1301 also has the same arrangement and functions as those of the data reading device 101 described in the first embodiment.

The antenna 1314 included in the antenna unit 1320, the conductor 1315 included in the running portion 1330, and the processing unit 1316 included in the body temperature measuring unit 1340 are integrally formed on the base sheet as a body temperature tag, and fixed between an observe film 1311 and a reserve film 1312 (each film is semipermeable and has a thickness of about 100 µm). Note that the antenna 1314, the conductor 1315, and the processing unit 1316 integrally formed on the base sheet will be referred to generically as a body temperature tag 1313 hereinafter.

Out of the observe film 1311 and the reserve film 1312, a film 1312b included in the antenna unit 1320 and the running portion 1330 can be obtained from materials including urethane-based polymers such as polyether urethane and polyester polyurethane, amide-based polymers such as a polyether polyamide block polymer, acrylic-based polymers such as polyacrylate, polyolefin-based polymers such as polyethylene, polypropylene, and an ethylene/vinyl acetate copolymer, and polyester-based polymers such as polyether polyester.

To prevent a skin surface with the film adhered from sweating or chlorosis, a film 1312a out of the reserve film 1312 included in the body temperature measuring unit 1340 is preferably selected from materials having water vapor permeability. For example, using an urethane- or amide-based film is suitable. Note that each of the observe film 1311 and the reserve film 1312b can use one of the above-described materials or be a laminated film formed by laminating a plurality of films made of arbitrary materials.

The reserve film 1312a has a thickness of 10 to 100 µm, and preferably, 20 to 40 µm to prevent any sense of incongruity when adhered to the skin surface. To make the film adhered to the skin surface excellently fit it, it is preferable to adjust the tensile strength to 100 to 900 kgf/cm² and the 100% modulus to about 10 to 100 kgf/cm². Using the reserve film 1312a adjusted within this range is effective when adhered to the skin surface that moves largely. From the viewpoint of preventing sweating, not only a non-porous film but also a porous film having water vapor permeability but no water permeability can effectively be used as the reserve film 1312a. Such a film can easily be obtained by a known porosification technique without particularly limiting the material. As the noticeable tendency of a non-porous film, the thicker the film is, the lower the water vapor permeability is. However, a porous film is useful because the water vapor permeability does not conspicuously lower in proportion to the thickness.

An adhesive is applied to the reserve film 1312a so that the clinical thermometer 1310 can directly be adhered to an appropriate measurement part on the body surface of an object. The adhesive can be any material of normal medical grade. Examples are acrylic-based adhesives, polyurethane-based adhesives, or natural rubber- or synthetic rubber-based adhesives, and solvent-, water borne-, hot melt-, and dry blend-based adhesives mainly containing a medical polymer. If radiation sterilization and, more particularly, intensive gamma-ray sterilization is necessary, it is preferable to avoid use of an acrylic-based adhesive or a polyurethane-based adhesive because radiation rays may lower the adhesion.

In addition, the observe film 1311 and the reserve film 1312a are so flexible that they can deform conforming to the shape of the measurement part of the object when the clinical thermometer 1310 is adhered to the measurement part. Since the processing unit 1316 is fixed in tight contact with the measurement part, the clinical thermometer 1310 can accurately detect the body temperature of the object.

Note that out of the antenna 1314, the conductor 1315, and the processing unit 1316 included in the body temperature tag 1313, the processing unit 1316 is covered by a heat insulator (for example, a heat insulator having a four-layer structure (nonwoven fabric + transparent polyethylene film + aluminum layer + polyethylene foam film) including an aluminum layer and formed into a thickness of about 1 mm). This enables to eliminate the influence of outside air temperature (ambient temperature).

On the other hand, the data reading device 1301 includes an RF-ID reader/writer. When approaching the body temperature tag 1313, the data reading device 1301 magnetically couples with it so as to supply power to the power supply circuit included in the processing unit 1316 of the body temperature tag 1313 and receive data from the body temperature tag 1313.

### <2. Body Temperature Measuring Method of Body Temperature Measuring System>

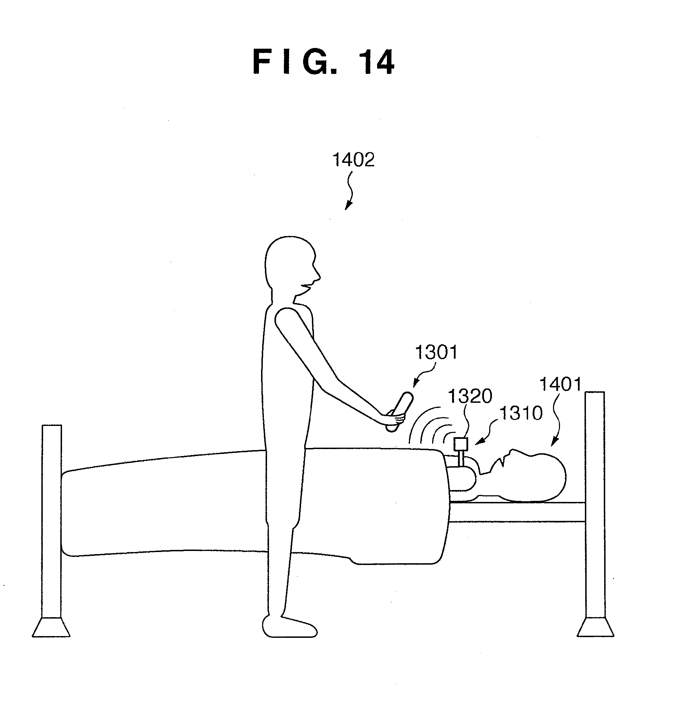

The body temperature measuring method of the body temperature measuring system 1300 will be described next. Fig. 14 illustrates a state in which the body temperature measuring unit 1340 of the clinical thermometer 1310 is attached to an axillary portion that is an appropriate measurement part of an object 1401. In the clinical thermometer 1310 with the body temperature tag according to this embodiment, the body temperature measuring unit 1340 and the antenna unit 1320 are connected via the running portion 1330. For this reason, even if the body temperature measuring unit 1340 is attached to the axillary portion of the object 1401, the antenna unit 1320 can be arranged apart from the axillary portion of the object 1401.

Hence, when an electromagnetic wave having a predetermined frequency of, for example, 13.56 MHz is generated at power level 1, and a measurer 1402 brings the data reading device 1301 closer to the clinical thermometer, the antenna 1314 can immediately be sensed, and electromagnetic coupling with the body temperature tag 1313 can easily and reliably be established at power level 2. That is, it is possible to prevent the problem of reading errors that may arise in the adhesive clinical thermometer with an antenna.

### <3. Steps in Manufacture of Clinical Thermometer 1310>

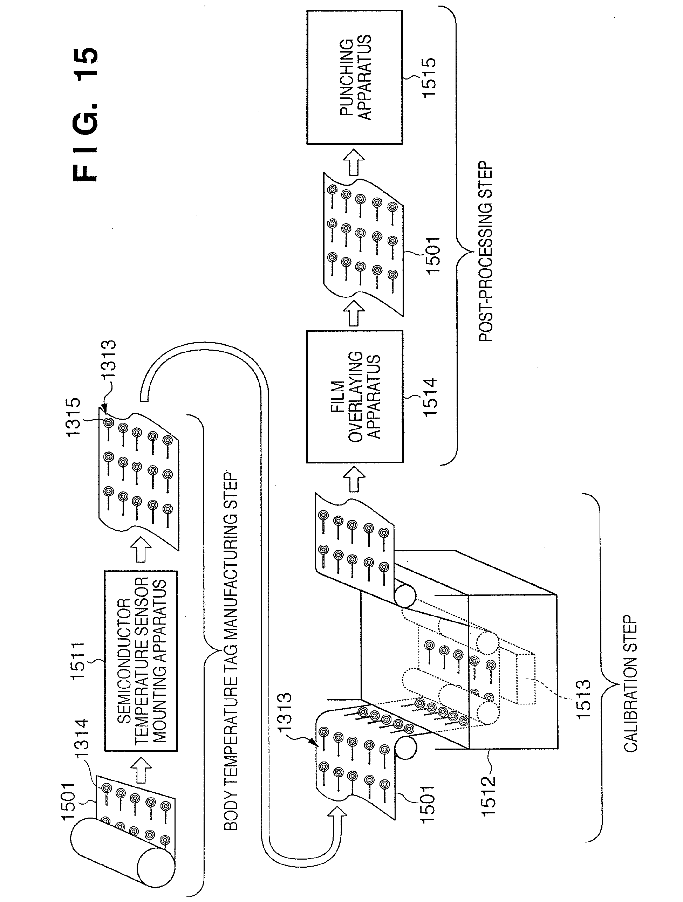

Steps in the manufacture of the clinical thermometer 1310 will be described next. Fig. 15 is a view showing the steps in the manufacture of the clinical thermometer 1310. Note that the manufacturing steps of the clinical thermometer 1310 are the same as in Fig. 11 except the shape of the body temperature tag 1313, and a description thereof will be omitted.

As is apparent from the above description, according to this embodiment, it is therefore possible to implement accurate body temperature measurement at a measurement accuracy within 0.05°C especially by obtaining a temperature resolution of 0.01°C at 32°C to 42°C that is the general measurement range of human body temperature measurement. In addition, data can reliably be read from the adhesive clinical thermometer with an antenna.

### [Third Embodiment]

In the second embodiment, the antenna unit 1320, the running portion 1330, and the body temperature measuring unit 1340 are arranged on the same plane. However, the present invention is not limited to this. For example, the antenna unit 1320 and the running portion 1330 may be arranged vertically with respect to the body temperature measuring unit 1340.

In the first embodiment, the antenna unit 1320, the running portion 1330, and the body temperature measuring unit 1340 are arranged to be bilaterally symmetrical. However, the present invention is not limited to this. For example, the antenna unit 1320 and the running portion 1330 may be arranged to be asymmetrical with respect to the body temperature measuring unit 1340.

In any case, the body temperature measuring unit 1340 preferably has a shape and size suitable for the measurement part of the object to which it is adhered. In addition, the shapes and sizes of the running portion 1330 and the body temperature measuring unit 1340 are preferably determined such that the antenna unit 1320 is arranged at a position where electromagnetic coupling with the data reading device 1301 is reliably ensured when the body temperature measuring unit 1340 is adhered to the measurement part of the object.

### [Fourth Embodiment]

In the first embodiment, when the value of digital data calculated by the control unit 205 is equal to or smaller than a predetermined value, it is determined that the body temperature tag is in the state that affects the body temperature measurement accuracy. The switch of the overheat preventing unit 201 is turned off to stop processing by the processing unit 115. However, the present invention is not limited to this.

For example, when the power supply voltage supplied via the antenna 114 is equal to or more than a predetermined voltage value, the processing unit 115 may determine that the body temperature tag is in the state that affects the body temperature measurement accuracy and forcibly turn off the switch.

In the first embodiment, the number of semiconductor temperature sensors parallelly connected in the sensor unit 211 has not specifically been mentioned. The number of parallelly connected semiconductor temperature sensors is preferably, for example, eight or so. If the number of parallelly connected semiconductor temperature sensors is small, the influence of the individual difference is large, and the measurement accuracy lowers. On the other hand, if the number of semiconductor temperature sensors is too large, the influence of errors due to heat generation is large.

In the first embodiment, the clinical thermometer 110 sends voltage data, calibration data, and identification information to the data reading device 101. However, the present invention is not limited to this. For example, when the measurement range is switched, information about the measurement range after the switching may be sent. In this case, the data reading device 101 calculates body temperature data in consideration of the received information about the measurement range as well.

Switching the measurement range may be done based on an instruction from the data reading device 101. In this case, the data reading device 101 calculates body temperature data in consideration of the designated measurement range.

The present invention is not limited to the above embodiments and various changes and modifications can be made within the spirit and scope of the present invention. Therefore, to apprise the public of the scope of the present invention, the following claims are made.

This application claims the benefit of Japanese Patent Application No. 2009-172563, filed July 23, 2009, which is hereby incorporated by reference herein in its entirety.

## Claims

1. A body temperature measuring system including a body temperature tag including an antenna unit and a processing unit, and a reading device for reading data from the body temperature tag, **characterized in that**
said processing unit of said body temperature tag comprises:
a power supply circuit connected to said antenna unit to be activated in accordance with generation of an induced electromotive force in said antenna unit;
detection means in which at least two semiconductor temperature sensors are connected parallel to each other, each of said semiconductor temperature sensors being formed by connecting two types of semiconductors including a p-type semiconductor and an n-type semiconductor and detecting a band gap voltage generated when a current is supplied to a connection portion between the two types of semiconductors; and
storage means for storing calibration data to calibrate the band gap voltage detected by said detection means, and
is configured to, upon activating said power supply circuit, send the band gap voltage detected by said detection means to said reading device via said antenna unit together with the calibration data,
said reading device comprises:
excitation means capable of exciting at a predetermined power level; and
sensing means for, when a magnetic field generated by excitation at a first power level by said excitation means has changed due to an influence of said antenna unit, sensing the change in the magnetic field, and
said excitation means is configured to, when said sensing means senses the change in the magnetic field, excite at a second power level that allows to generate, in said antenna unit, the induced electromotive force to activate said power supply circuit.

2. A data reading device that electromagnetically couples with a body temperature tag including an antenna unit and a processing unit,
said processing unit comprising:
a power supply circuit connected to said antenna unit to be activated in accordance with generation of an induced electromotive force in said antenna unit;
detection means in which at least two semiconductor temperature sensors are connected parallel to each other, each of said semiconductor temperature sensors being formed by connecting two types of semiconductors including a p-type semiconductor and an n-type semiconductor and detecting a band gap voltage generated when a current is supplied to a connection portion between the two types of semiconductors; and
storage means for storing calibration data to calibrate the band gap voltage detected by said detection means, and
being configured to, upon activating said power supply circuit, send the band gap voltage detected by said detection means via said antenna unit together with the calibration data, the device **characterized by** comprising:
excitation means capable of exciting at a predetermined power level; and
sensing means for, when a magnetic field generated by excitation at a first power level by said excitation means has changed due to an influence of said antenna unit, sensing the change in the magnetic field,
wherein said excitation means is configured to, when said sensing means senses the change in the magnetic field, excite at a second power level that allows to generate, in said antenna unit, the induced electromotive force to activate said power supply circuit.

3. The system according to claim 1, **characterized in that** said detection means comprises six to 10 semiconductor temperature sensors and detects an average value of band gap voltages at the connection portions of said semiconductor temperature sensors.

4. The system according to claim 1, **characterized in that** said detection means comprises eight semiconductor temperature sensors and detects an average value of band gap voltages at the connection portions of said semiconductor temperature sensors.

5. The system according to claim 1, **characterized in that** said detection means detects an average value of band gap voltages when the current is supplied to the connection portion of each semiconductor temperature sensor a plurality of number of times.

6. The system according to claim 1, **characterized in that**
said detection means further comprises boost means for boosting the induced electromotive force generated in said antenna unit,
and the current is supplied to the connection portion of each semiconductor temperature sensor based on a voltage boosted by said boost means.

7. The system according to claim 1, **characterized in that** said processing unit further comprises stop means for stopping processing performed upon activation of said power supply circuit if a temperature rises upon generation of the induced electromotive force in said antenna unit.

8. The system according to claim 1, **characterized in that** said processing unit further comprises switching means for switching the detected band gap voltage to a band gap voltage in a predetermined range.

9. A driving control method of a data reading device that electromagnetically couples with a body temperature tag including an antenna unit and a processing unit,
the processing unit comprising:
a power supply circuit connected to the antenna unit to be activated in accordance with generation of an induced electromotive force in the antenna unit;
detection means in which at least two semiconductor temperature sensors are connected parallel to each other, each of the semiconductor temperature sensors being formed by connecting two types of semiconductors including a p-type semiconductor and an n-type semiconductor and detecting a band gap voltage generated when a current is supplied to a connection portion between the two types of semiconductors; and
storage means for storing calibration data to calibrate the band gap voltage detected by the detection means, and
being configured to, upon activating the power supply circuit, send the band gap voltage detected by the detection means via the antenna unit together with the calibration data, the method **characterized by** comprising:
the excitation step capable of exciting at a predetermined power level; and
the sensing step of, when a magnetic field generated by excitation at a first power level in the excitation step has changed due to an influence of the antenna unit, sensing the change in the magnetic field,
wherein in the excitation step, when the change in the magnetic field is sensed in the sensing step, excitation is performed at a second power level that allows to generate, in the antenna unit, the induced electromotive force to activate the power supply circuit.
